# EUROPEAN PATENT SPECIFICATION

(11) **EP 2 129 405 B1**
(45) Date of publication and mention of the grant of the patent: **25.05.2011**
(21) Application number: 08705523.2
(22) Date of filing: 08.01.2008
(51) Int. Cl.: A61L 2/08

(54) **PACKAGING FOR PRODUCTS TO BE DECONTAMINATED BY RADIATION**
VERPACKUNG FÜR PRODUKTE, DIE DURCH STRAHLUNG DEKONTAMINIERT WERDEN
EMBALLAGE POUR PRODUITS DEVANT ÊTRE DÉCONTAMINÉ PAR RAYONNEMENT

(30) Priority: 09.01.2007 FR 0700120
(43) Date of publication of application: 09.12.2009
(73) Proprietor: Becton, Dickinson and Company, Franklin Lakes, NJ 07417 (US)
(72) Inventor: PEROT, Frederic, 38760 Saint-Paul-de-Varces (FR)
(74) Representative: Maureau, Philippe
(86) International application number: PCT/US2008/000255
(87) International publication number: WO 2008/085969

(56) References cited:
- WO-A-02/40064
- WO-A-02/40065

## Description

### BACKGROUND OF THE INVENTION

### 1. Field of the Invention

The invention relates to a packaging for products that is to be decontaminated by radiation.

The conditions of sterility in which certain stages of the handling or transportation of products or instruments intended for medical use are to be performed are extremely strict, particularly in the pharmaceutical industry. It is, therefore, extremely important to produce packaging compatible with such requirements.

### 2. Description of the Related Art

In the present application, the expression "selectively impervious material" is to be understood as meaning that the material is designed, in terms of structure, to control any exchange between the inside of the packaging and its external environment. This means, among other things, that the packaging is impervious to contamination by micro-organisms, bacteria and/or a biologically active material likely to come into contact with the packaging while it is being handled, while at the same time remaining permeable to a sterilization or decontamination by radiation or gas, for example of the ETO (ethylene oxide) type.

In the present application, the expression "screen against electron radiation" is to be understood as being a screen capable of reflecting and/or partially or completely absorbing the kinetic energy of the electrons from an electron beam, and therefore of slowing or even preventing these electrons from passing through the screen.

Packagings for products that are or may be sterilized comprising a tub sealed with a cover sheet made of a selectively impervious material are known.

Usually, to proceed with the sterilization of the products contained in such packaging, a sterilization gas, for example of the ethylene oxide type, enters the tub through the sheet of selectively impervious material. The tub containing the sterilized products is then placed in a protective bag so that the tub can be transported. By way of example, such a tub or packaging may contain syringes intended to be filled with a drug in a sterile room or controlled-environment room. It is also possible for the packaging to be placed beforehand in a protective bag which has a window closed with a material that is permeable to the sterilization gas, and for the sterilization to be subsequently performed on the closed bag.

To proceed with the subsequent handling step, for example the filling of the syringes, the protective bag needs to be opened. The packaging, which may then be contaminated, needs to be decontaminated before it is taken, for example, into a sterile room. Such decontamination can be achieved using multidirectional irradiation by an electron beam developing enough energy that when it has passed through the cover sheet, it delivers a dose of irradiation of, for example, 25 kGy. This means that it can be taken that the selectively impervious material has been decontaminated throughout its thickness, particularly the sealed zone, at the interface between the tub and the material. As far as the rest of the packaging is concerned, the combination of the density and thickness of the packaging is such that it stops these electrons.

This type of decontamination is not, however, suitable for every type of product transported in the packaging. This is because the electron beam passing through the sheet of selectively impervious material carries the risk of altering or adversely affecting the material of which the syringes or products placed in the tub are made, for example glass. The electron beam can also generate ozone from the oxygen in the air contained in the tub. The generated ozone carries the risk of polluting the atmosphere and of adversely affecting the active products used to fill the syringes and/or, for example, the rubber components present in the tub such as the caps on the needles mounted on the syringes.

To try to solve these problems, some packaging have been proposed, that include a screen against electron radiation.

Document WO02/40064 discloses a packaging comprising a tub sealed with a cover sheet made of a selectively impervious material, the packaging containing syringe bodies. The packaging disclosed in WO02/40064 also comprises a screen against electron radiation, with the screen located inside the tub.

Nevertheless, such a packaging may not be totally satisfactory. In particular, there is a need for a simple packaging easy to manufacture and to handle during the numerous steps of its use.

One embodiment of the present invention aims at satisfying this need by providing a packaging for products so that an electron beam can be used to carry out biological decontamination of this packaging at any time during the different handling steps of the packaging, with the packaging also being very easy to manipulate.

### SUMMARY OF THE INVENTION

The present invention relates to a packaging according to claim 1.

The packaging of the invention is easy to manufacture and to handle. Moreover, the packaging of the invention can be easily decontaminated, at any time of its use, without any risk for the products contained inside the packaging.

According to the invention, one of the two layers has its shape and/or dimension different from the shape and/or dimension of the other layer.;

In an embodiment of the invention, the first layer has a first peripheral outline, the second layer has a second peripheral outline, the cover sheet has a third peripheral outline, the first layer being fixed to the second layer along at least part of the first peripheral outline, the second layer being fixed to the cover sheet along at least part of the second peripheral outline and the cover sheet being fixed to the tub along the third peripheral outline, two of the first, second and third peripheral outlines being different.

In an embodiment of the invention, said first peripheral outline is comprised in the area defined by the second peripheral outline which is itself comprised in the area defined by the third peripheral outline.

In an embodiment of the invention, the packaging comprises an additional filter located above the products inside said tub.

In an embodiment of the invention, the screen is attached to the cover sheet by bonding or welding.

The screen may comprise at least one layer of a material made of filaments of polymer bound together by heat and pressure. Preferably, the polymer is a high density polyethylene.

In an embodiment of the invention, the products are medical devices.

### BRIEF DESCRIPTION OF THE DRAWINGS

Other features and advantages will become apparent from the detailed description given hereinafter, given by way of example with reference to the appended drawings in which:

Figure 1 is cross section view of a packaging-which is not an embodiment of the invention,

Figure 2 is a perspective view of the packaging of figure 1,

Figure 3 is a schematic partial cross section view of an embodiment of a packaging which is not of the invention,

Figure 4 is a schematic partial cross section view of an embodiment of the packaging of the invention, and

Figure 5 is a schematic partial cross section view of an embodiment of a packaging which is not of the invention.

### DETAILED DESCRIPTION OF THE PRESENTLY PREFERRED

### EMBODIMENTS

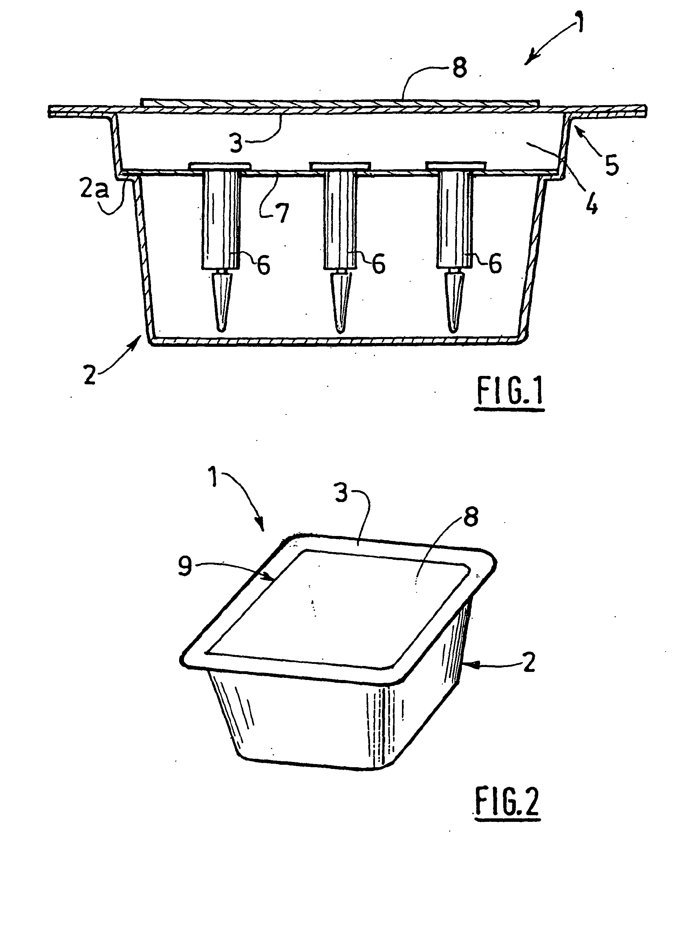

Figure 1 shows a packaging 1 which is not of the invention comprising a tub 2 and a cover sheet 3. The tub 2 may be made out of plastics material. The tub 2 has an opening 4 and a peripheral zone 5 defined about the opening 4. The cover sheet 3 is fixed to the peripheral zone 5 of the tub 2 so as to seal the opening 4 imperviously. The cover sheet 3 is made of a selectively impervious material. The selectively impervious material may be a material made of filaments of a high density polyethylene bound together by heat and pressure, such as the product sold by the Company Du Pont under the trademark "TYVEK®".

The packaging 1 comprises products, under the form of medical devices such as syringe bodies 6 in the example shown, that are sterilized or to be sterilized. On the example shown on figure 1, the syringe bodies 6 are received in holes designed on a plate 7, the plate 7 being placed inside the tub 2, bearing on a rim 2a provided on the inner wall of the tub 2. On top of the cover sheet 3, outside of the tub 2, is placed a screen 8 against the electron radiation. The screen 8 is dimensioned to substantially cover the syringe bodies 6 and may be made of one layer of filaments of a high density polyethylene bound together by heat and pressure, such as the product sold by the Company Du Pont under the trademark "TYVEK®".

As shown on figure 2, the screen 8 against electron radiation defines a peripheral outline 9. The screen 8 against electron radiation may be attached to the cover sheet 3, for example along all or part of the peripheral outline 9, by bonding or welding. The packaging 1 is thus very easy to manufacture and to manipulate during the different steps of its use.

In an alternative embodiment not shown, the peripheral outline 9 of the screen has a shape different from the one of the peripheral zone 5 of the tub 2.

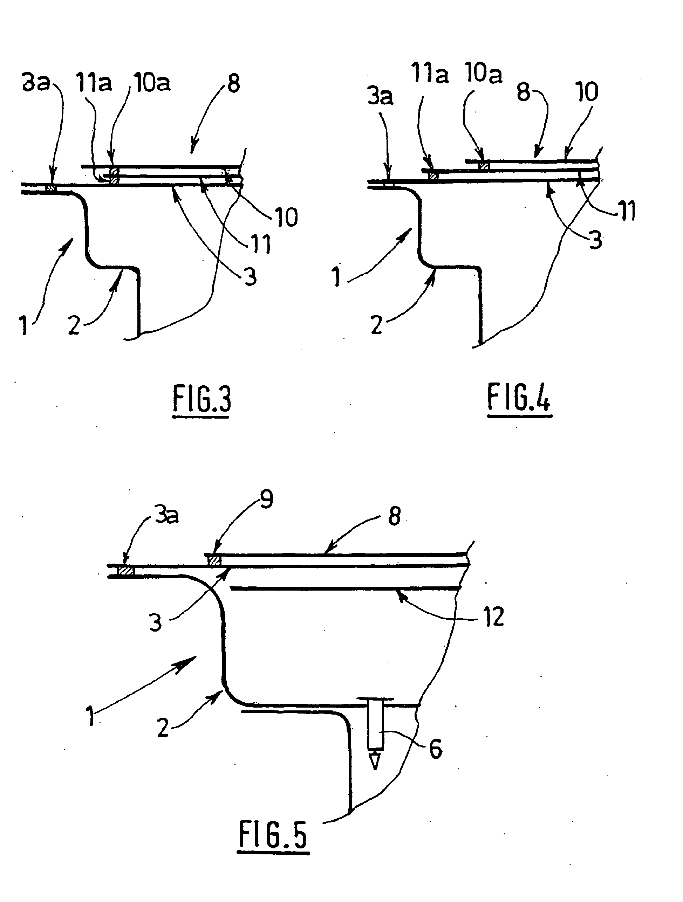

Figure 3 and 4 are schematic partial cross section views of respectively an alternative embodiment which is not of the invention and an embodiment of the invention: on these figures, for sake of simplicity, the products such as the syringes bodies 6 in figures 1 and 2 have not been represented.

In an embodiment which is not of the invention shown on figure 3, the screen 8 comprises two layers, a first layer 10 and a second layer 11, of material fixed on the cover sheet 3. The material may be made of filaments of a high density polyethylene bound together by heat and pressure. On the example shown on figure 3, the two layers (10, 11) have similar shapes and dimensions. The the two layers of material may be attached one to the other, for example by means of gluing, bonding or welding, preferably along part of all their respective peripheral outlines.

On figure 3, the first layer 10 has a first peripheral outline 10a, the second layer 11 has a second peripheral outline 11a, and the cover sheet 3 has a third peripheral outline 3a. The first layer 10 is fixed to the second layer 11 along the first peripheral outline 10a, the second layer 11 is fixed to the cover sheet 3 along the second peripheral outline 11a and the cover sheet 3 is fixed to the tub 2 along the third peripheral outline 3a. As appears clearly on this figure, the first and second peripheral outline (10a, 11a) are similar with each other and different from the third peripheral outline 3a.

Figure 4 shows a variant of the packaging 1 of figure 3 which is in accordance with the invention, in which the first layer 10 has a shape and dimensions different from the second layer 11. The references designating the same elements as in figure 3 have been preserved. In the example shown in figure 4, the first peripheral outline 10a, the second peripheral outline 11a and the third peripheral outline 3a are all different. In this embodiment, the second peripheral outline 11a is more easily accessible than in the previous embodiment of figure 3 and ensures a more reliable decontamination of the area located along second peripheral outline 11a.

In another embodiment which is not of the invention shown on figure 5, the packaging 1 of the invention further comprises an additional filter 12 located above the syringe bodies 6 inside the tub 2. This additional filter 12 may be a screen against electron radiation. For instance, the additional filter 12 may be made of one layer of filaments of a high density polyethylene bound together by heat and pressure, such as the product sold by the Company Du Pont under the trademark "TYVEK®".

The packaging of the invention may be efficiently decontaminated by being submitted to electron beam without altering the integrity of the products it contains. The products contained in the packaging of the invention are not affected by the electron radiation applied on the packaging, in particular towards the cover sheet during decontamination of the packaging. The packaging of the invention is particularly easy to handle and to manipulate. Indeed, the screen can be attached to the cover sheet before the use of the cover sheet to seal the tub opening.

## Claims

1. Packaging (1) for products, that is to be decontaminated by radiation, comprising:
at least one tub (2) having an opening and a peripheral zone defined about said opening;
a cover sheet (3) made of a selectively impervious material and fixed to the peripheral zone of the tub so as to seal said opening imperiously; and
at least one screen (8) against electron radiation dimensioned to substantially cover said products, said screen comprising a first layer (10) and a second layer (11), of material fixed on said cover sheet, **characterized in that** said screen being located on top of said cover sheet, outside of the tub, one of the two layers of said screen has its shape and/or dimension different from the shape and/or dimension of the other layer of said screen.

2. The packaging according to claim 1, wherein said first layer (10) has a first peripheral outline (10a), said second layer (11) has a second peripheral outliner (11a), said cover sheet (3) has a third peripheral outline (3a), said first layer being fixed to the second layer along at least part of said first peripheral outline, said second layer being fixed to said cover sheet along at least part of said second peripheral outline and said cover sheet, two of said first, second and third peripheral outlines being different.

3. The packaging according to claim 2, wherein said first peripheral outline (10a) is comprised in the area defined by said second peripheral outline (11a) which is itself comprised in the area defined by said third peripheral outlines (3a).

4. The packaging according to any one of claims 1 to 3, wherein said packaging comprises a filter located above said products inside said tub.

5. The packaging according to claim 1, wherein said screen is attached to said cover sheet by bonding or welding.

6. The packaging according to claim 5, wherein said screen comprises at least one layer of a material made of filaments of polymer bound together by heat and pressure.

7. The packaging according to claim 6, wherein said polymer is a high density polyethylene.

8. The packaging according to claim 1 or 2, wherein said products are medical devices.

## Patentansprüche

1. Verpackung (1) für Produkte, die es durch Strahlung zu dekontaminieren gilt, umfassend:
zumindest eine Wanne (2) mit einer Öffnung und einem peripheren Bereich, der um die Öffnung herum definiert ist;
eine Abdeckfolie (3), die aus selektiv undurchlässigem Material hergestellt und am peripheren Bereich der Wanne befestigt ist, um die Öffnung undurchlässig abzudichten; und
zumindest einen Schirm (8) gegen Elektronenstrahlung, der derart dimensioniert ist, um die Produkte im Wesentlichen abzudecken, wobei der Schirm eine erste Schicht (10) und eine zweite Schicht (11) aus Material, das an der Abdeckfolie befestigt ist, umfasst, **dadurch gekennzeichnet, dass** der Schirm oben außerhalb der Wanne auf der Abdeckfolie platziert ist, wobei sich die Gestalt und/oder die Dimension einer der beiden Schichten des Schirms von der Gestalt und/oder der Dimension der anderen Schicht des Schirms unterscheidet bzw. unterscheiden.

2. Verpackung nach Anspruch 1, wobei die erste Schicht (10) einen ersten peripheren Umriss (10a) aufweist, wobei die zweite Schicht (11) einen zweiten peripheren Umriss (11a) aufweist, wobei die Abdeckfolie (3) einen dritten peripheren Umriss (3a) aufweist, wobei die erste Schicht an der zweiten Schicht entlang zumindest eines Teils des ersten peripheren Umrisses befestigt ist, wobei die zweite Schicht an der Abdeckfolie entlang zumindest eines Teils des zweiten peripheren Umrisses und der Abdeckfolie befestigt ist, wobei zwei des ersten, des zweiten und des dritten Umrisses unterschiedlich sind.

3. Verpackung nach Anspruch 2, wobei der erste periphere Umriss (10a) im Bereich umfasst ist, der durch den zweiten peripheren Umriss (11a) definiert ist, der selbst im Bereich umfasst ist, der vom dritten peripheren Umriss (3a) definiert ist.

4. Verpackung nach einem der Ansprüche 1 bis 3, wobei die Verpackung einen Filter umfasst, der über den Produkten innerhalb der Wanne positioniert ist.

5. Verpackung nach Anspruch 1, wobei der Schirm durch Kleben oder Schweißen an der Abdeckfolie befestigt ist.

6. Verpackung nach Anspruch 5, wobei der Schirm zumindest eine Schicht áus einem Materials umfasst, das aus Polymerfilamenten hergestellt ist, die durch Hitze und Druck miteinander verbunden sind.

7. Verpackung nach Anspruch 6, wobei das Polymer ein Polyethylen mit hoher Dichte ist.

8. Verpackung nach Anspruch 1 oder 2, wobei es sich bei den Produkten um medizinische Geräte handelt.

## Revendications

1. Emballage (1) pour produits, destiné à être décontaminé par rayonnement, comprenant au moins une boîte (2) ayant une ouverture et une zone périphérique définie autour de ladite ouverture, une feuille de couverture (3) constituée d'un matériau sélectivement étanche et fixée à la zone périphérique de la boîte de manière à sceller ladite ouverture de manière étanche, au moins un écran (8) contre le rayonnement d'électrons dimensionné de manière à recouvrir substantiellement lesdits produits, ledit écran comprenant une première couche (10) et une deuxième couche (11), de matériau fixées sur ladite feuille de couverture, **caractérisé en ce que** ledit écran étant placé au-dessus de ladite feuille de couverture, à l'extérieur de la boîte, l'une des deux couches dudit écran a une forme et/ou dimension différente(s) de la forme et/ou dimension de l'autre couche dudit écran.

2. Emballage selon la revendication 1, dans lequel ladite première couche (10) a un premier contour périphérique (10a), ladite deuxième couche (11) a un deuxième contour périphérique (11a), ladite feuille de couverture (3) a un troisième contour périphérique (3a), ladite première couche étant fixée à la deuxième couche le long d'au moins une partie dudit premier contour périphérique, ladite deuxième couche étant fixée à ladite feuille de couverture le long d'au moins une partie dudit deuxième contour périphérique et ladite feuille de couverture étant fixée à la boîte le long dudit troisième contour périphérique, deux desdits premier, deuxième et troisième contours périphériques étant différents.

3. Emballage selon la revendication 2, dans lequel ledit premier contour périphérique (10a) est compris dans la zone définie par ledit deuxième contour périphérique (11a), qui est lui-même compris dans la zone définie par ledit troisième contour périphérique (3a).

4. Emballage selon l'une quelconque des revendications 1 à 3, dans lequel ledit emballage comprend un filtre situé au-dessus desdits produits à l'intérieur de ladite boîte.

5. Emballage selon la revendication 1, dans lequel ledit écran est fixé à ladite feuille de couverture par fixation ou soudage.

6. Emballage selon la revendication 5, dans lequel ledit écran comprend au moins une couche d'un matériau constitué de filaments de polymère liés conjointement par la chaleur et la pression.

7. Emballage selon la revendication 6, dans lequel ledit polymère est un polyéthylène haute densité.

8. Emballage selon la revendication 1 ou 2, **caractérisé en ce que** lesdits produits sont des dispositifs médicaux.
